# EUROPEAN PATENT SPECIFICATION

(11) **EP 0 626 826 B1**
(45) Date of publication and mention of the grant of the patent: **02.10.2002**
(21) Application number: 93901158.1
(22) Date of filing: 22.12.1992
(51) Int. Cl.: A61B 18/20

(54) **HANDPIECE FOR TRANSMYOCARDIAL VASCULARIZATION HEART-SYNCHRONIZED PULSED LASER SYSTEM**
HANDSTÜCK FÜR EIN HERZSYNCHRONIESIERTES LASERSYSTEM ZUR TRANSSYNCHROCARDIALEN VASCULARISATION
PIECE A MAIN DESTINEE A LA REVASCULARISATION TRANSMYOCARDIQUE PAR SYSTEME LASER PULSE SYNCHRONISE PAR RAPPORT AU COEUR

(43) Date of publication of application: 07.12.1994
(73) Proprietor: PLC MEDICAL SYSTEMS, INC., Milford, MA 01757 (US)
(72) Inventor: RUDKO, Robert, I., Holliston, MA 01746 (US)
(74) Representative: Bowles, Sharon Margaret
(86) International application number: US9211002
(87) International publication number: WO94014383

(56) References cited:
- JP-A- 55 153 908
- US-A- 3 865 113
- US-A- 3 913 582
- US-A- 4 266 548
- US-A- 4 757 515
- US-A- 4 850 352
- US-A- 4 940 411

## Description

### FIELD OF INVENTION

This invention relates to a handpiece for use in a transmyocardial vascularization heart-synchronized pulsed laser system, and more particularly to such a handpiece whose contact surface avoids destabilization of the beating heart.

### BACKGROUND OF INVENTION

Transmyocardial revascularization (TMR) is an alternative technique to bypass surgery for increasing blood flow to the heart muscle. It involves the puncturing of the heart wall with a laser to form a plurality of holes which heal on the outside but remain open on the inside of the heart, to provide an alternative source of blood to the heart muscle. This technique has been employed on a stilled, by-passed heart using a CO₂ laser with a hand-piece which rests on the heart in order to ensure that the laser beam focus occurs at the correct point on the heart. Recently, a dramatic improvement in TMR has enabled this technique to be used on a beating heart without the need to slow or still it. This has been accomplished with an innovative synchronizing approach. However, this has introduced new problems. A beating heart is electrically active; the contact of a handpiece against the heart wall disrupts that electrical activity and interferes with the heart function. Arrhythmia and fibrillation can occur and can result in heart failure. Further, any interference with the electrical field of the heart interrupts the synchronous operation of the laser so that the laser is no longer constrained to fire at the optimum moment in the beating heart cycle. The current handpieces used with CO₂ lasers have a relatively sharp tip on a gauge rod extending from the end of the handpiece used to consistently position the handpiece at the proper distance from the stilled heart wall for accurate laser beam focusing and impingement. Such a tip creates increased pressure on the heart, which can cuase arrhythmia, fibrillation, and can even puncture the wall of the heart. Further, with these handpieces it is difficult to maintain the laser beam perpendicular with the wall of a beating heart as is necessary to effect clean, correctly placed holes in the heart wall.

US specification 4,850,352 discloses a laser-surgical instrument with a barrel, a passage for transmitting the laser beam, a focusing system and an evacuation tip. A sheath surrounds the tip and is spaced apart from it to allow passage of purge gas. The sheath can be extended beyond the tip to permit evacuation.

### SUMMARY OF THE INVENTION

According to the invention there is provided a handpiece for use in a pulsed laser surgical system comprising: means for focusing a laser beam and a barrel having a passage for transmitting the laser beam, the barrel terminating at a distal end having an aperture for exit of the laser beam characterised in that the distal end of the barrel has an end surface surrounding the aperture adapted for stable contact with a beating heart in a heart-synchronised transmyocardial revascularisation procedure, said end surface being smooth, flat and extending continuously from the aperture so as to provide a stable contact platform for maintaining perpendicularity between the beam and the heart.

The means for focusing may comprise a lens or lens unit that enables the laser to be focused at, near or beyond the aperture.

It is therefore an object of this invention to provide an improved laser handpiece for a heart-synchronized pulsed laser system for transmyocardial vascularization.

It is a further object of this invention to provide such a laser handpiece which more readily maintains perpendicularity with the wall of a beating heart.

It is a further object of this invention to provide such a laser handpiece which accurately locates the laser beam focal point at the correct point on the heart wall.

It is a further object of this invention to provide such a laser handpiece which reduces interference with the heart electric field and function.

It is a further object of this invention to provide such a laser handpiece which dissipates the laser plume to prevent interference with or damage to the laser beam lens.

This invention results from the realization that an effective and safe handpiece capable of contacting the wall of a beating heart to insure proper protection and focus of the laser beam, yet minimize danger to or interference with the beating heart can be achieved by focusing the laser beam in the vicinity of the laser beam exit aperture at the end of the handpiece and providing a large, smooth, flat heart contact surface at that end of the handpiece to minimize pressure on and interference with the beating heart.

In a preferred embodiment the handpiece further includes means for introducing a gas to purge the passage, between the aperture and the means for focusing, of debris from the vaporized heart wall. There may also be exhaust means for venting the debris purged by the gas. The barrel may be straight or may be angled and include deflecting means for redirecting the laser beam along the angled barrel. The deflecting means may include a mirror. The contact surface at the distal end of the barrel is generally smooth and flat with round edges, and is generally greater than 1cm in diameter. The gas may be introduced proximate the means for focusing and exhausted proximate the aperture. The beam may be focused beyond the enlarged surface, within the barrel or intermediately within the aperture.

### DISCLOSURE OF A PREFERRED EMBODIMENT

Other objects, features and advantages will occur to those skilled in the art from the following description of a preferred embodiment and the accompanying drawings, in which:
Fig. 1 is a three-dimensional view of a CO₂ surgical laser system employing the handpiece of this invention;
Fig. 2 is an enlarged view of a handpiece according to this invention and a portion of the articulated optical arm which carries it;
Fig. 3 is an enlarged cross-sectional view of the focusing lens section of the handpiece of Figs. 1 and 2;
Fig. 4 is an enlarged sectional view of the barrel of the handpiece of Figs. 1 and 2; and
Fig. 5 is a side elevational view with portions broken away of an alternative form of barrel similar to that shown in Fig. 4.

The handpiece of this invention for use in a transmyocardial revascularization heart-synchronized pulsed laser system may be accomplished using a barrel having a passage for transmitting a laser beam. The barrel may be simply a hollow tube. There is a surface at the distal end of the barrel for contacting the wall of the heart. This surface is smooth and flat so that there are no sharp edges to probe or prick the heart wall. It is also broad in order to minimize the contact pressure between the handpiece and the heart wall and minimize interference with the operation of the heart muscle and the electrical activity of the beating heart. The handpiece, at least at its contact surface, is electrically and thermally insulating for the same purpose. There is an aperture located at the distal end of the barrel in the enlarged surface for transmitting a laser beam through to the heart wall. There are also some means for focusing the laser beam proximate to the aperture to vaporize the tissue of the heart wall and create a hole through the wall to the interior of the heart chamber. The laser may be focused at, near or beyond the aperture. There is an inlet to introduce a purging gas through the passage to purge, the aperture and the means for focusing, of debris produced by the vaporization of the heart wall by the laser beam. The means for focusing is typically a lens which is mounted in the focusing unit or lens unit associated with the barrel. There is one or more outlets proximate the distal end of the barrel through which the purged gas with the debris is vented. The barrel may be straight or may be angled. If it is argled there are suitable deflecting means such as mirrors or reflectors, to redirect the beam along the angled or curved barrel. Typically the enlarged surface for contacting the heart is 1 cm or more in diameter.

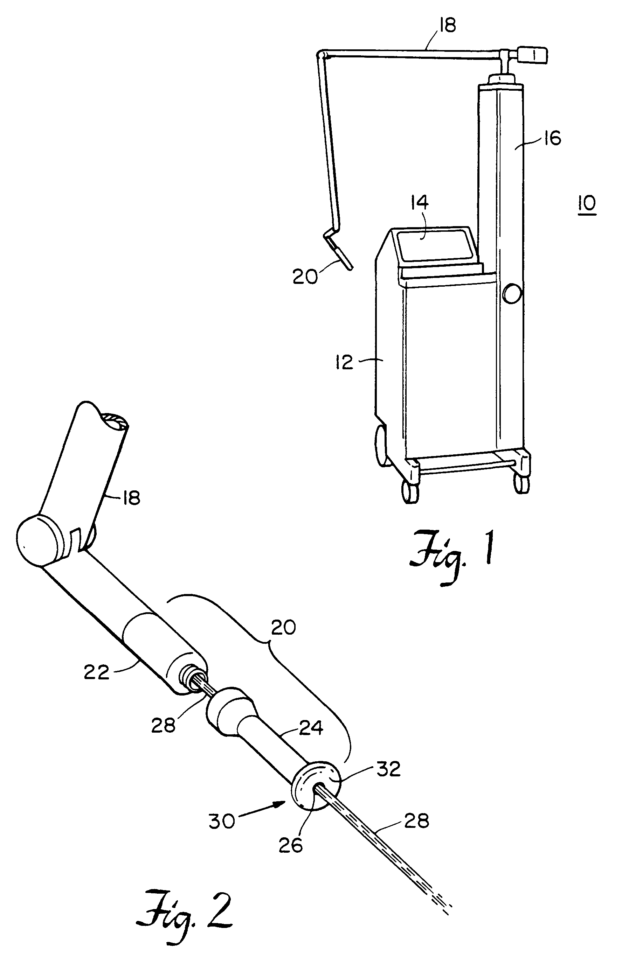

There is shown in Fig. 1 a surgical laser system 10 including a power supply 12 and control panel 14 for operating CO₂ laser 16, whose output beam is directed through@@ F articulated arm 18 to handpiece 20. Handpiece 20 may include a lens unit 22 including a lens for focusing the laser beam and a barrel 24 which includes an aperture 26 through which the laser beam 28 exits. The distal end 30 of barrel 24 includes an enlarged contact surface 32 for contacting the wall of the heart to be perforated by the laser beam. Surface 32 is relatively large to minimize the contact pressure between it and the heart wall, and is flat and smooth with rounded edges to minimize interference with the heart. Surface 32 is typically 1 cm or greater in diameter, and may be electrically and thermally insulating.

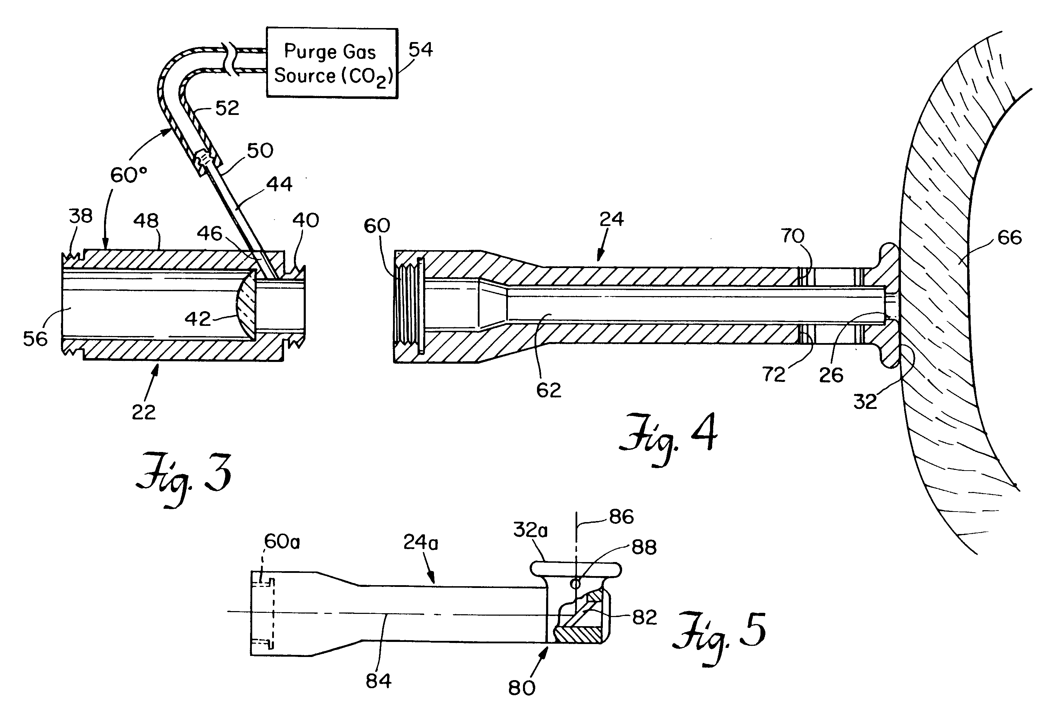

The focusing unit or lens unit 22, Fig. 3, includes a threaded portion 38 for interconnection with arm 18, and a threaded portion 40 which interconnects with barrel 24. Carried within unit 22 is focusing lens 42. An inlet tube 44 is joined by interference fit with bore 46 and a cylindrical wall 48 of unit 22. At its free end 50, inlet 44 is connected to a hose 52 which is in turn connected to a purge gas source 54 which provides a gas such as CO₂ under gentle pressure to create a backflow from lens 42 forward into barrel 24. This keeps any debris from the vaporization from contacting and obscuring or damaging lens 42. Lens 42 is positioned directly in line with passage 56 provided in unit 22 for propagation of the laser beam. Threads 40 of lens unit 22 engage with threads 60 of barrel 24, Fig. 4, which also includes a passage 62 which communicates with laser aperture 26 to create a clear passage for the propagation of laser beam 20a to wall 66 of a beating heart. Lens 42 focuses the laser beam proximate aperture 26 and surface 32.

As can be seen clearly in Fig. 4, contact surface 32 is considerably broader than the cross-sectional area of barrel 24 alone and is formed in the shape of a flange with surface 32 being smooth and flat and all the edges rounded. This increases the area of contact with the heart, and therefore decreases the pressure or force per unit area on the heart. It also provides a more stable platform by which to maintain perpendicularity between the beam 28 and the heart wall 66. Thus this construction provides the necessary precision in locating the focus of the beam on the heart wall without interfering with the heart operation or its electrical activity. Barrel 24 includes vent holes 70, 72 for exhausting the purging gas and trapped debris away from the lens 42 and away from aperture 26.

Although barrel 24 has been shown as a straight member, this is not a necessary limitation of the invention. For example, barrel 24a, Fig. 5, may include a right angle configuration 80, so that surface 32a is facing at right angles to the path of the beam 28. A reflective surface 82 is provided to reflect the beam from an incoming path parallel to axis 84 to the outgoing path parallel to axis 86. One or more vent holes 88 are provided for exhausting the first gas.

Although specific features of the invention are shown in some drawings and not others, this is for convenience only as each feature may be combined with any or all of the other features in accordance with the invention.

Other embodiments will occur to those skilled in the art and are within the following claims:

## Claims

1. A handpiece for use in a pulsed laser surgical system comprising:
means for focusing (22) a laser beam and a barrel (24) having a passage (62) for transmitting the laser beam, the barrel terminating at a distal end (30) having an aperture (26) for exit of the laser beam **characterised in that** the distal end of the barrel has an enlarged end surface (32) surrounding the aperture adapted for stable contact with a beating heart in a heart-synchronised transmyocardial revascularisation procedure, said enlarged end surface being smooth, flat and extending continuously from the aperture so as to provide a stable contact platform for maintaining perpendicularity between the beam and the heart, and the means for focusing is adapted to focus the laser beam proximate the aperture to vapourise the tissue of the heart wall and create a hole to the interior heart chamber.

2. A handpiece according to claim 1 in which the enlarged end surface (32) is thermally insulating.

3. A handpiece according to claim 1 or claim 2 in which the means for focusing comprises a lens or lens unit that enables the laser to be focused at, near or beyond the aperture.

4. A handpiece according to any preceding claim further including means (44) for introducing a gas to purge the passage (42) between the aperture and the means for focusing of debris produced by vaporisation of the heart wall.

5. A handpiece according to claim 4 further including exhaust means (70, 72) in communication with the passage (62) for venting debris purged by the gas.

6. A handpiece according to claim 5 in which the exhaust means (70, 72) is located proximate the aperture (26).

7. A handpiece according to any preceding claim in which the end surface (32) is greater than 0.01 metres in diameter.

8. A handpiece according to any preceding claim in which the end surface (32) is in the form of a flange extending from the barrel (24) and has rounded edges.

9. A handpiece according to any preceding claim in which the barrel (24) is straight.

10. A handpiece according to any of claims 1 to 8 in which the barrel (24) is angled and includes deflecting means (80) for directing the laser beam along the angled barrel.

11. A handpiece according to claim 10 in which the deflecting means includes a mirror (82).

## Patentansprüche

1. Handstück für die Verwendung in einem chirurgischen System mit gepulstem Laser, umfassend:
Mittel zum Fokussieren (22) eines Laserstrahls und einen Zylinder (24) mit einem Durchgang (62) zum Übertragen des Laserstrahls, wobei der Zylinder an einem distalen Ende (30) endet, das eine Öffnung (26) für den Austritt des Laserstrahls aufweist, **dadurch gekennzeichnet, dass** das distale Ende des Zylinders eine vergrößerte Endfläche (32) aufweist, die die Öffnung umgibt und die für einen stabilen Kontakt mit einem schlagenden Herz in einem Herz-synchronisierten transmyokardialen Revaskularisationsverfahren angepasst ist, wobei die vergrößerte Endfläche glatt, flach ist und sich stetig von der Öffnung erstreckt, so dass eine stabile Kontaktplattform bereitgestellt wird, um eine lotrechte Stellung zwischen dem Strahl und dem Herzen zu halten, und wobei die Mittel zum Fokussieren angepasst sind, um den Laserstrahl unmittelbar an der Öffnung zu fokussieren, um das Gewebe der Herzwand zu verdampfen und ein Loch in der inneren Herzkammer zu erzeugen.

2. Handstück nach Anspruch 1, wobei die vergrößerte Endfläche (32) wärmeisolierend ist.

3. Handstück nach Anspruch 1 oder 2, wobei die Mittel zum Fokussieren eine Linse oder eine Linseneinheit umfassen, die es dem Laser ermöglicht, bei, nahe an der Öffnung oder über die Öffnung hinaus fokussiert zu werden.

4. Handstück nach einem der vorstehenden Ansprüche, das ferner Mittel (44) zum Einleiten eines Gases einschließt, um den Durchgang (42) zwischen der Öffnung und den Mitteln zum Fokussieren von Trümmern zu spülen, die durch die Verdampfung der Herzwand erzeugt worden sind.

5. Handstück nach Anspruch 4, das ferner Entlüftungsmittel (70, 72) in Kommunikation mit dem Durchgang (62) einschließt, um die durch das Gas gespülten Trümmer abzuleiten.

6. Handstück nach Anspruch 5, wobei die Entlüftungsmittel (70, 72) unmittelbar an der Öffnung (26) angeordnet sind.

7. Handstück nach einem der vorstehenden Ansprüche, wobei die Endfläche (32) größer als 0,01 m im Durchmesser ist.

8. Handstück nach einem der vorstehenden Ansprüche, wobei die Endfläche (32) in Form eines Flansches vorliegt, der sich von dem Zylinder (24) erstreckt, und abgerundete Kanten aufweist.

9. Handstück nach einem der vorstehenden Ansprüche, wobei der Zylinder (24) gerade ist.

10. Handstück nach einem der Ansprüche 1 bis 8, wobei der Zylinder (24) winkelig ist und Ablenkmittel (80) einschließt, um den Laserstrahl entlang dem winkeligen Zylinder zu lenken.

11. Handstück nach Anspruch 10, wobei die Ablenkmittel einen Spiegel (82) einschließen.

## Revendications

1. Pièce à main destinée à être utilisée dans un système chirurgical à laser pulsé, comportant :
un moyen pour focaliser (22) un faisceau laser et un corps (24) ayant un passage (62) pour transmettre le faisceau laser, le corps aboutissant à une extrémité distale (30) ayant une ouverture (26) pour la sortie du faisceau laser, **caractérisée en ce que** l'extrémité distale du corps présente une surface extrême élargie (32) entourant l'ouverture et conçue pour établir un contact stable avec un coeur qui bat dans une procédure de revascularisation transmyocardique synchronisée avec le coeur, ladite surface extrême élargie étant lisse, plate et s'étendant en continu depuis l'ouverture afin de procurer une plate-forme de contact stable pour maintenir la perpendicularité entre le faisceau et le coeur, et le moyen de focalisation est conçu pour focaliser le faisceau laser à proximité de l'ouverture afin de vaporiser les tissus de la paroi du coeur et de créer un trou vers la chambre cardiaque intérieure.

2. Pièce à main selon la revendication 1, dans laquelle la surface extrême élargie (32) est thermiquement isolante.

3. Pièce à main selon la revendication 1 ou la revendication 2, dans laquelle le moyen de focalisation comporte une lentille ou un bloc à lentilles qui permet au laser d'être focalisé à l'ouverture, à proximité de celle-ci ou au-delà de celle-ci.

4. Pièce à main selon l'une quelconque des revendications précédentes, comprenant en outre un moyen (44) pour l'introduction d'un gaz pour purger le passage (42), entre l'ouverture et le moyen de focalisation, de débris produits par la vaporisation de la paroi cardiaque.

5. Pièce à main selon la revendication 4, comprenant en outre un moyen d'évacuation (70, 72) en communication avec le passage (62) pour évacuer les débris purgés par le gaz.

6. Pièce à main selon la revendication 5, dans laquelle le moyen d'évacuation (70, 72) est placé à proximité de l'ouverture (26).

7. Pièce à main selon l'une quelconque des revendications précédentes, dans laquelle la surface extrême (32) a un diamètre supérieur à 0,01 mètre.

8. Pièce à main selon l'une quelconque des revendications précédentes, dans laquelle la surface extrême (32) se présente sous la forme d'une collerette s'étendant depuis le corps (24) et présente des bords arrondis.

9. Pièce à main selon l'une quelconque des revendications précédentes, dans laquelle le corps (24) est droit.

10. Pièce à main selon l'une quelconque des revendications 1 à 8, dans laquelle le corps (24) est coudé et comprend un moyen de déviation (80) destiné à diriger le faisceau laser le long du corps coudé.

11. Pièce à main selon la revendication 10, dans laquelle le moyen de déviation comprend un miroir (82).
